# EUROPEAN PATENT SPECIFICATION

(11) **EP 4 154 919 B1**
(45) Date of publication and mention of the grant of the patent: **08.04.2026**
(21) Application number: 22197677.2
(22) Date of filing: 26.09.2022
(51) Int. Cl.: A61L 9/14, A61L 9/03, A61L 9/12

(54) **SCENT DISPENSING SYSTEM**
DUFTABGABESYSTEM
SYSTÈME DE DISTRIBUTION DE PARFUM

(30) Priority: 27.09.2021 US 202163248959 P
(43) Date of publication of application: 29.03.2023
(73) Proprietor: Chimera Technology LLC, Palatine, IL 60067 (US)
(72) Inventor: Bruder, Geoffrey A., Rocky River, OH, 44116 (US); Flood, David, Palatine, IL, 60067 (US)
(74) Representative: Tansini, Elio Fabrizio

(56) References cited:
- WO-A1-2016/164917
- WO-A1-2018/022562
- WO-A1-2019/151948
- JP-B2- 6 929 349
- US-A1- 2020 078 485

## Description

### FIELD OF THE DISCLOSURE

The present disclosure relates to a scent dispensing system, and more particularly to a scent dispensing system for Cannabinoid containing products.

### BACKGROUND

The 2018 Farm Bill legalized the sale of cannabinoid containing products in the United States provided that Delta-9 THC content was kept below 0.3% in the plant and product. Cannabinoid containing products are becoming ubiquitous in the market and the variety of products available are difficult for consumers to understand. Educating the consumer about the process of production, from seed to sale, as well as informing them about the chemical compounds within the product is a challenge due to the tactile nature of the end product. It would be advantageous to allow customers to smell cannabinoid containing products before deciding to purchase them. A need therefore exists for a scent dispensing system having a user interface that allows a user to choose a Cannabinoid containing product and be provided with the scent of the chosen product.

### BRIEF SUMMARY OF THE INVENTION

The invention relates to a scent dispensing system comprising a plurality of storage tanks containing a liquid medium of terpenes, scent agents, and/or flavoring agents, and one or more dispensing devices that are adapted to deliver atomized liquid medium from the plurality of storage tanks to produce a scent selection for a user. Each of the one or more dispensing devices further comprises a vapor generator connected to a supply of water, wherein each vapor generator is adapted to deliver a visible mist of water vapor along with the atomized liquid.

In a further aspect of the invention, a scent dispensing system comprises a plurality of storage tanks containing a liquid medium of terpenes, scent agents, and/or flavoring agents, and one or more dispensing devices that are adapted to deliver atomized liquid medium from the plurality of storage tanks to produce a scent selection for a user. One or more pumps are disposed between the plurality of storage tanks and the one or more dispensing devices, wherein thee one or more pumps are adapted to pump the liquid medium from the storage tanks to the one or more dispensing devices.

### BRIEF DESCRIPTION OF THE DRAWINGS

FIG. 1 shows a scent dispensing system, according to an embodiment;

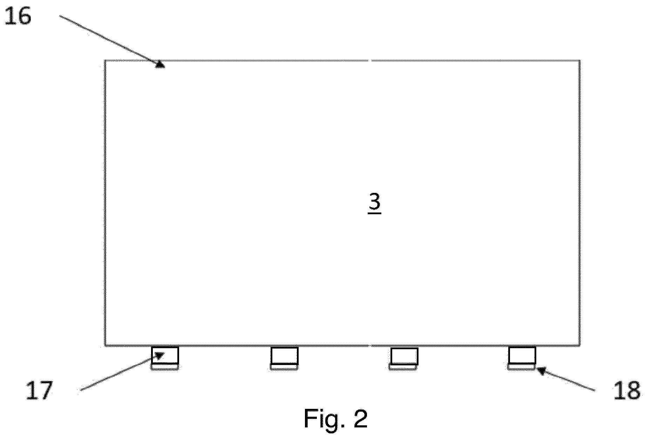

FIG. 2 shows a schematic representation of a user interface, according to an embodiment;.
FIG. 3 shows a photograph of an exemplary scent dispensing system, according to an embodiment;
FIG. 4 shows an exemplary dispensing device, according to an embodiment;
FIG. 5 shows a scent dispensing system, according to another embodiment; and
FIG. 6 shows a scent dispensing system, according to a further embodiment.

### DETAILED DESCRIPTION

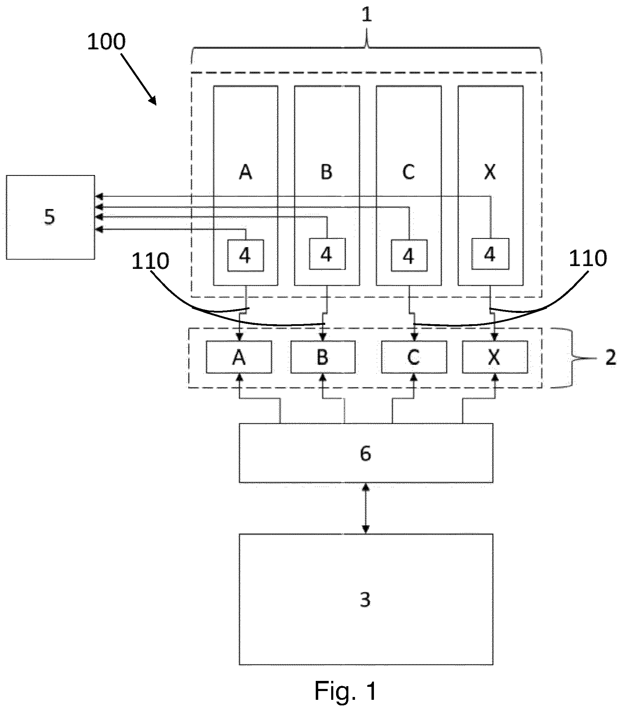

Referring to FIG. 1, an embodiment of a scent dispensing system 100 comprises a plurality of storage tanks 1 (that are individually labeled as A, B, C, X). Each of the plurality of storage tanks 1 holds a liquid medium, for example without limitation, comprising terpenes, and/or other scent and flavoring agents that mimic the smell of a desired component to be delivered by the system 100. In an embodiment the liquid medium may also comprise a thinning agent, for example without limitation water, an oil, or an alcohol, wherein the thinning agent enables the concentration of the final delivered liquid to be adjusted. In an embodiment the plurality of storage tanks 1 is made of a corrosion resistant material, for example without limitation stainless steel, aluminum, glass, or polyvinylchloride (PVC), wherein each of the storage tanks 1 is sealed against leaking.

In an embodiment each of the plurality of storage tanks 1 is connected to a dispensing device 2 (having individual receiving modules labeled as A, B, C, X) either directly or through corrosion resistant connections 110, wherein the connections 110 can be made, for example without limitation, from stainless steel, aluminum, protective lined Tygon^{®}, or PVC tubing. In an embodiment all of the plurality of storage tanks 1 connect to a single dispensing device 2; however, in other embodiments the plurality of storage tanks 1 connects to a plurality of dispensing devices 2.

Still referring to FIG. 1, in an embodiment a system controller 6 is in electrical communication with and receives input data from a user interface 3. In an embodiment the system controller 6 is in electrical communication with and transmits control signals generated from the input data to the one or more dispensing devices 2. Electrical communication for data transfer between any of the components described herein can be via a wired connection or via a wireless connection using any wireless protocol as is known in the art.

In an embodiment the system controller 6 stores data regarding interactions with the user interface 3, for example input data regarding the selection of product scents to sample. In an embodiment the system controller 6 is connected to the internet allowing remote monitoring and/or troubleshooting of the system controller 6, and further allowing software used by the system controller 6 to reside on a cloud-based platform and be deployed to multiple system controllers 6 within multiple scent dispensing systems 100.

Still referring to FIG. 1, in an embodiment the user interface 3 is an analog interface, for example, comprising buttons and/or switches. In an embodiment the user interface 3 is a digital interface, for example, comprising a touchscreen. In an embodiment the user interface 3 is a combination analog and digital interface.

In an embodiment the user interface 3 conveys information to a user about one or more chemical compounds to be delivered via the one or more dispensing devices 2. In an embodiment the user interface 3 allows a user to select which compound they would like to smell and select or toggle a selection input which in turn sends a signal to the system controller 6.

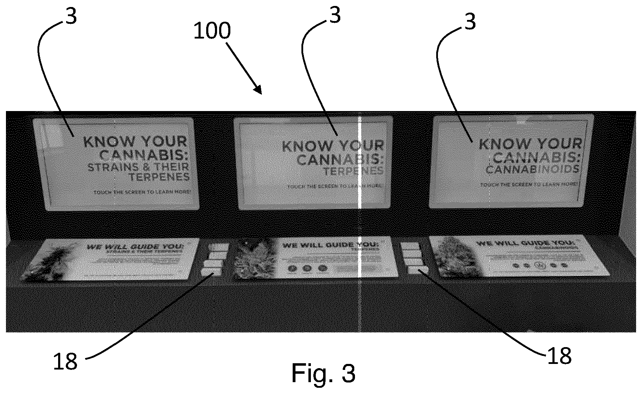

Referring to FIG. 3, in an embodiment a user interface 3 comprises a touchscreen 16, a plurality of dispensers blocks 17 and a plurality of dispensing ports 18. Each dispensing port 18 is the physical location where a user will smell and see vaporized fluid delivered from the plurality of storage tanks 1. FIG. 3 shows a photograph of an installation of an exemplary scent dispensing system 100 having three user interfaces 3 and eight dispensing ports 18.

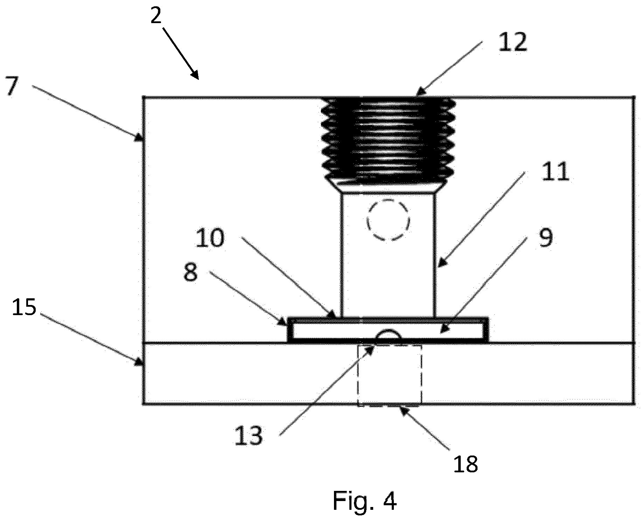

In an embodiment the one or more dispensing devices 2 deliver a scent from the storage tanks 1 to a user. Along with the scent, in an embodiment a visible atomization cloud is generated. Referring to FIG. 4, in an embodiment a main block 7 comprises an interface between the storage tanks 1 and an atomization device 9. In an embodiment the main block 7 is made of a corrosion resistant material such as stainless steel, aluminum, or PVC. In an embodiment the main block 7 comprises a storage tank connection 12, transport passage 11, atomizer cavity 8, and one or more mounting point (not shown). In an embodiment the storage tank connection 12 is a threaded connection, hose barb, push connection, or the like, which fluidly connects the main block 7 to a storage tank 1 while remaining leak-proof. In another embodiment the storage tank connection 12 and the transport passage 11 are part of a tube or hose or pipe (see 110 in FIG. 1) that connects the storage tanks 1 to the one or more dispensing devices 2.

Still referring to FIG. 4, in an embodiment the transport passage 11 allows liquid to flow from the storage tank connection 12 to the atomizer cavity 8 at which point the liquid contacts the atomization device 9. In an embodiment the atomizer cavity 8 provides a volume which securely contains the atomization device 9 and an atomizer seal 10. In an embodiment the atomizer seal 10 is a malleable material that, for example, provides a liquid tight seal around the atomization device 9 when compressed between the main block 7 and a clamp block 15.

In an embodiment the clamp clock 15 is secured to the main block 7, for example, by being bolted, clamped or otherwise affixed in a manner that maintains compression of the atomizer seal 10 throughout operation, but is removable to allow for servicing. In an embodiment the clamp block 15 further comprises a dispensing port 18 which allows for vaporized liquid to exit the atomization device 9 and reach the user. In an embodiment an electrical connection port 13 is contained wholly within the main block 7, within the clamp block 15, or partially within both. In an embodiment the electrical connection port 13 allows for wiring from the system controller 6 to reach the atomization device 9. In an embodiment the atomization device 9 is piezoelectric; however, in other embodiments the atomization device 9 is pneumatic, or another sort of liquid atomization device that is electrically actuated.

Referring to FIGS. 1, 5, and 6, in an embodiment, within each storage tank 1 is disposed a level sensor 4, which is electronically connected to a level control unit 5. In an embodiment the level sensor 4 within a storage tank 1 transmits an electrical signal to the level control unit 5 when the liquid within the storage tank 1 reaches a low setpoint, whereupon the level control unit 5 then communicates an alarm to an operator. In an embodiment the level control unit 5 is integrated into the system controller 6; however, in another embodiment the level control unit 5 is a stand-alone unit. Furthermore, in an embodiment the level control unit 5 records data to determine which liquid is most frequently utilized.

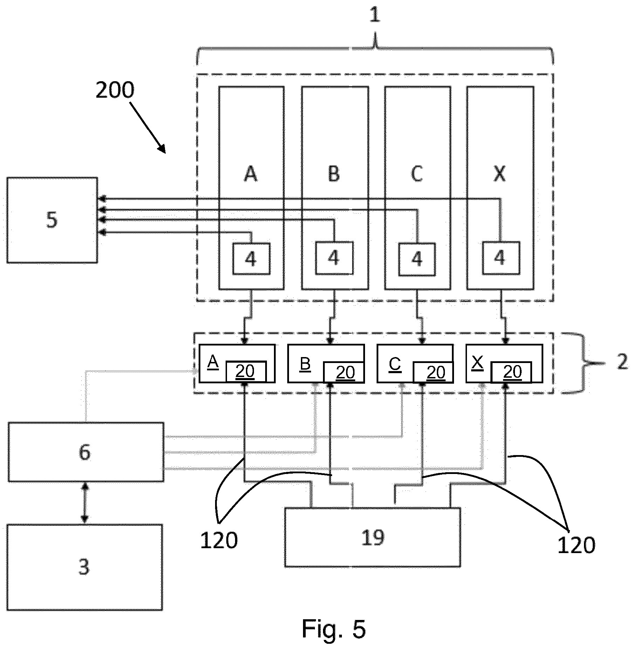

Referring to FIG. 5, an embodiment of a scent dispensing system 200 includes a water tank 19 and a vapor generator 20. In an embodiment the water tank 19 is a container made of glass, PVC, or another metal or plastic that resists corrosion with prolonged exposure to water. In an embodiment the water tank 19 is connected to the vapor generator 20 by flexible or rigid tubing 120 that is metal or plastic that resists biological buildup and corrosion with prolonged exposure to water. In an embodiment the water tank 19 is disposed above the vapor generator 20 such that gravity allows water to flow into the vapor generator 20 when actuated. In an embodiment the water lines 120 from the water tank 19 may connect in series to all the vapor generators 20 rather than by individual connections so long as the vapor generators 20 are successively lower in elevation, thereby allowing gravity to feed them in succession. In an embodiment a valve (not shown) may be placed at the lowest and highest points within the water lines 120 to allow air to be bled from the water lines 120. In an embodiment as a user selects a compound to smell, the system controller 6 sends a signal to the vapor generator 20, which releases a visible mist of water vapor utilizing water from the water tank 19 along with the desired scent, by actuating the appropriate dispensing device 2.

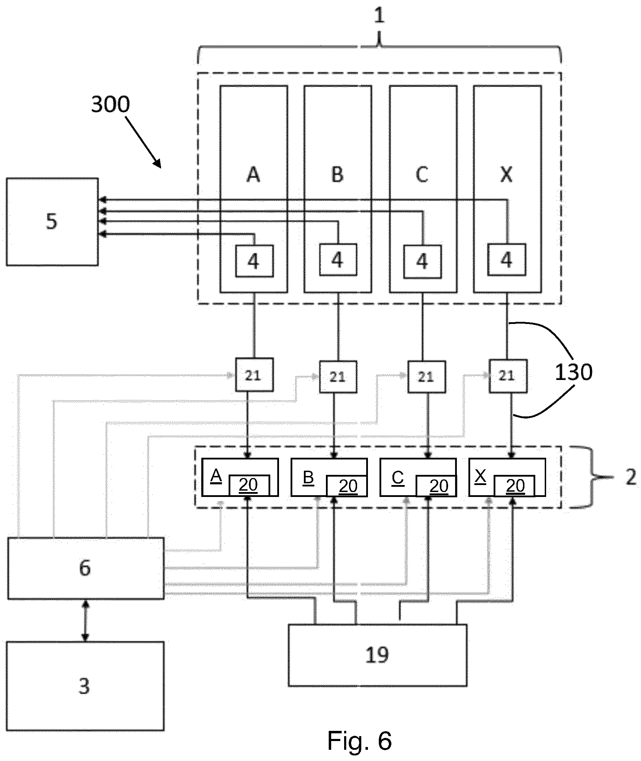

Referring to Figure 6, an embodiment of a scent dispensing system 300 includes a plurality of pumps 21, for example without limitation, chemical resistant pumps 21 such as a diesel fuel transfer pump, in the scent dispensing path. In an embodiment the plurality of pumps 21 is connected to between the plurality of storage tanks 1 and the one or more dispensing devices 2 through corrosion resistant connections130, wherein the connections 130 can be made, for example without limitation, from stainless steel, aluminum, protective lined Tygon^{®}, or PVC tubing. In an embodiment the plurality of pumps 21 is controlled, electrically, via the system controller 6.

Upon a user selecting a scent via the user interface 3, in an embodiment the system controller 6 sends an electrical signal to the plurality of pumps 21, which pumps the scent containing liquid medium from the plurality of storage tanks 1 to the one or more dispensing devices 2. In an embodiment that utilizes the plurality of pumps 21, the plurality of storage tanks 1 can be disposed below the one or more dispensing devices 2 to move the liquid medium against gravity.

As will be realized, the present disclosure is capable of other and different embodiments, and its several details are capable of modifications in various obvious respects, all without departing from the scope of the claims. Accordingly, the drawings and description are to be regarded as illustrative in nature, and not as restrictive.

### INDUSTRIAL APPLICABILITY

A scent dispensing system comprises a plurality of storage tanks containing a liquid medium of terpenes, scent agents, and/or flavoring agents, and one or more dispensing devices that are adapted to deliver atomized liquid medium from the plurality of storage tanks to produce a scent selection for a wherein each of the one or more dispensing devices further comprises a vapor generator connected to a supply of water, wherein each vapor generator is adapted to a visible mist of water vapor along with the atomized liquid. In an embodiment one or more pumps are disposed between the plurality of storage tanks and the one or more dispensing devices, wherein the one or more pumps are adapted to pump the liquid medium from the storage tanks to the one or more dispensing devices. The scent dispensing system can be manufactured in industry for use by consumers.

Numerous modifications to the present invention will be apparent to those skilled in the art in view of the foregoing description. It is not desired to limit the invention to the exact construction and operation shown and described, and accordingly, all suitable modifications and equivalents may be resorted to, falling within the scope of the appended claims. Accordingly, this description is to be construed as illustrative only of the appended claims and is presented for the purpose of enabling those skilled in the art to make and use the invention and to teach the best mode of carrying out same. The exclusive rights to all modifications which come within the scope of the appended claims are reserved.

## Claims

1. A scent dispensing system (100), comprising:
a plurality of storage tanks (1), each storage tank containing a liquid medium selected from the group of media consisting of terpenes, scent agents, flavoring agents, and combinations thereof; and
one or more dispensing devices (2) that are adapted to deliver atomized liquid medium from the plurality of storage tanks (1);
wherein each of the one or more dispensing devices (2) further comprises a vapor generator (20) connected to a supply of water, wherein each vapor generator (20) is adapted to deliver a visible mist of water vapor along with the atomized liquid medium.

2. The scent dispensing system (100) of claim 1, further comprising a user interface (3) adapted to receive a scent selection from a user, and a system controller (6) electrically connected to the user interface (3) and to the one or more dispensing devices (2), wherein the system controller (6) receives input data regarding the scent selection and transmits control signals to the one or more dispensing devices (2).

3. The scent dispensing system (100) of claim 2, wherein the user interface (3) is a digital interface including a touchscreen (16).

4. The scent dispensing system (100) of claim 2, wherein the system controller (6) stores data regarding interactions with the user interface (3).

5. The scent dispensing system (100) of claim 2, wherein the system controller (6) is internet connected.

6. The scent dispensing system (100) of claim 1, wherein each of the one or more dispensing devices (2) comprises a dispensing port (18) through which the atomized liquid medium is dispensed.

7. The scent dispensing system (100) of claim 1, wherein each of the one or more dispensing devices (2) comprises an electrically actuated pneumatic atomization device (9).

8. The scent dispensing system (100) of claim 1, wherein the liquid medium further comprises a thinning agent for adjusting a concentration of the liquid medium.

9. The scent dispensing system (100) of claim 8, wherein the thinning agent is selected from the group consisting of water, an oil, an alcohol, and combinations thereof.

10. The scent dispensing system (100) of claim 1, wherein the plurality of storage tanks (1) is constructed of a corrosion resistant material and is sealed against leaking.

11. The scent dispensing system (100) of claim 10, wherein the corrosion resistant material is selected from the group of materials consisting of stainless steel, aluminum, and polyvinylchloride (PVC).

12. The scent dispensing system (100) of claim 1, wherein the plurality of storage tanks (1) includes at least one level sensor (4), which is electronically connected to a level control unit (5) and records levels of the liquid medium within each of the plurality of storage tanks (1) to determine which liquid medium is most frequently utilized.

13. The scent dispensing system (100) of claim 1, further comprising:
one or more pumps (21) disposed between the plurality of storage tanks (1) and the one or more dispensing devices (2), wherein the one or more pumps (21) are adapted to pump the liquid medium from the storage tanks (1) to the one or more dispensing devices (2).

14. scent dispensing system (100) of claim 1, wherein each of the plurality of storage tanks (1) is connected to a respective one of the one or more dispensing devices (2) via a tube (110, 130).

## Patentansprüche

1. Duftabgabesystem (100), umfassend:
eine Vielzahl von Vorratsbehältern (1), wobei ein jeder Vorratsbehälter ein flüssiges Medium enthält, das aus der Gruppe von Medien ausgewählt ist, die aus Terpenen, Duftstoffen, Aromastoffen und Kombinationen davon besteht; und
eine oder mehrere Abgabevorrichtungen (2), die angepasst sind, um zerstäubtes flüssiges Medium aus der Vielzahl von Vorratsbehältern (1) abzugeben;
wobei eine jede der einen oder mehreren Abgabevorrichtungen (2) ferner einen Dampferzeuger (20) umfasst, der mit einer Wasserversorgung verbunden ist,
wobei ein jeder Dampferzeuger (20) angepasst ist, um einen sichtbaren Wasserdampfnebel zusammen mit dem zerstäubten flüssigen Medium abzugeben.

2. Duftabgabesystem (100) nach Anspruch 1, ferner umfassend eine Benutzerschnittstelle (3), die angepasst ist, um eine Duftauswahl von einem Benutzer zu empfangen, und eine Systemsteuerung (6), die elektrisch mit der Benutzerschnittstelle (3) und mit der einen oder den mehreren Abgabevorrichtungen (2) verbunden ist, wobei die Systemsteuerung (6) Eingabedaten bezüglich der Duftauswahl empfängt und Steuersignale an die eine oder die mehreren Abgabevorrichtungen (2) überträgt.

3. Duftabgabesystem (100) nach Anspruch 2, wobei die Benutzerschnittstelle (3) eine digitale Schnittstelle ist, die einen Touchscreen (16) einschließt.

4. Duftabgabesystem (100) nach Anspruch 2, wobei die Systemsteuerung (6) Daten bezüglich Interaktionen mit der Benutzerschnittstelle (3) speichert.

5. Duftabgabesystem (100) nach Anspruch 2, wobei die Systemsteuerung (6) mit dem Internet verbunden ist.

6. Duftabgabesystem (100) nach Anspruch 1, wobei eine jede der einen oder mehreren Abgabevorrichtungen (2) eine Abgabeöffnung (18) umfasst, durch die das zerstäubte flüssige Medium abgegeben wird.

7. Duftabgabesystem (100) nach Anspruch 1, wobei eine jede der einen oder mehreren Abgabevorrichtungen (2) eine elektrisch betätigte pneumatische Zerstäubungsvorrichtung (9) umfasst.

8. Duftabgabesystem (100) nach Anspruch 1, wobei das flüssige Medium ferner ein Verdünnungsmittel zum Einstellen einer Konzentration des flüssigen Mediums umfasst.

9. Duftabgabesystem (100) nach Anspruch 8, wobei das Verdünnungsmittel aus der Gruppe ausgewählt ist, die aus Wasser, einem Öl, einem Alkohol und Kombinationen davon besteht.

10. Duftabgabesystem (100) nach Anspruch 1, wobei die Vielzahl von Vorratsbehältern (1) aus einem korrosionsbeständigen Material gebaut ist und gegen Auslaufen abgedichtet ist.

11. Duftabgabesystem (100) nach Anspruch 10, wobei das korrosionsbeständige Material aus der Gruppe von Materialien ausgewählt ist, die aus rostfreiem Stahl, Aluminium und Polyvinylchlorid (PVC) besteht.

12. Duftabgabesystem (100) nach Anspruch 1, wobei die Vielzahl von Vorratsbehältern (1) mindestens einen Füllstandssensor (4) einschließt, der elektronisch mit einer Füllstandssteuereinheit (5) verbunden ist und Füllstände des flüssigen Mediums in einem jeden der Vielzahl von Vorratsbehältern (1) aufzeichnet, um zu bestimmen, welches flüssige Medium am häufigsten verwendet wird.

13. Duftabgabesystem (100) nach Anspruch 1, ferner umfassend:
eine oder mehrere Pumpen (21), die zwischen der Vielzahl von Vorratsbehältern (1) und der einen oder den mehreren Abgabevorrichtungen (2) angeordnet sind, wobei die eine oder mehreren Pumpen (21) dazu angepasst sind, das flüssige Medium von den Vorratsbehältern (1) zu der einen oder den mehreren Abgabevorrichtungen (2) zu pumpen.

14. Duftabgabesystem (100) nach Anspruch 1, wobei ein jeder der Vielzahl von Vorratsbehältern (1) über einen Schlauch (110, 130) mit einer jeweiligen der einen oder mehreren Abgabevorrichtungen (2) verbunden ist.

## Revendications

1. Système de distribution de parfum (100), comprenant :
une pluralité de réservoirs de stockage (1), chaque réservoir de stockage contenant un milieu liquide choisi parmi le groupe de milieux constitué de terpènes, d'agents de parfum, d'agents aromatisants et de leurs combinaisons ; et
un ou plusieurs dispositifs de distribution (2) qui sont adaptés pour délivrer un milieu liquide atomisé à partir de la pluralité de réservoirs de stockage (1) ;
dans lequel chacun des un ou plusieurs dispositifs de distribution (2) comprend en outre un générateur de vapeur (20) relié à une alimentation en eau, dans lequel chaque générateur de vapeur (20) est adapté pour délivrer un brouillard visible de vapeur d'eau avec le milieu liquide atomisé.

2. Système de distribution de parfum (100) selon la revendication 1, comprenant en outre une interface utilisateur (3) adaptée pour recevoir une sélection de parfum de la part d'un utilisateur, et un contrôleur de système (6) relié électriquement à l'interface utilisateur (3) et à l'un ou plusieurs dispositifs de distribution (2), dans lequel le contrôleur de système (6) reçoit des données d'entrée concernant la sélection de parfum et transmet des signaux de commande aux un ou plusieurs dispositifs de distribution (2).

3. Système de distribution de parfum (100) selon la revendication 2, dans lequel l'interface utilisateur (3) est une interface numérique incluant un écran tactile (16).

4. Système de distribution de parfum (100) selon la revendication 2, dans lequel le contrôleur de système (6) stocke des données concernant des interactions avec l'interface utilisateur (3).

5. Système de distribution de parfum (100) selon la revendication 2, dans lequel le contrôleur de système (6) est connecté à Internet.

6. Système de distribution de parfum (100) selon la revendication 1, dans lequel chacun des un ou plusieurs dispositifs de distribution (2) comprend un orifice de distribution (18) à travers lequel le milieu liquide atomisé est distribué.

7. Système de distribution de parfum (100) selon la revendication 1, dans lequel chacun des un ou plusieurs dispositifs de distribution (2) comprend un dispositif d'atomisation pneumatique (9) à actionnement électrique.

8. Système de distribution de parfum (100) selon la revendication 1, dans lequel le milieu liquide comprend en outre un fluidifiant pour ajuster une concentration du milieu liquide.

9. Système de distribution de parfum (100) selon la revendication 8, dans lequel le fluidifiant est choisi parmi le groupe constitué d'eau, d'une huile, d'un alcool et de leurs combinaisons.

10. Système de distribution de parfum (100) selon la revendication 1, dans lequel la pluralité de réservoirs de stockage (1) est construite en un matériau résistant à la corrosion et est scellée pour éviter les fuites.

11. Système de distribution de parfum (100) selon la revendication 10, dans lequel le matériau résistant à la corrosion est choisi parmi le groupe de matériaux constitué de l'acier inoxydable, de l'aluminium et du polychlorure de vinyle (PVC).

12. Système de distribution de parfum (100) selon la revendication 1, dans lequel la pluralité de réservoirs de stockage (1) inclut au moins un capteur de niveau (4), qui est relié électroniquement à une unité de contrôle de niveau (5) et enregistre les niveaux du milieu liquide dans chacun des réservoirs de la pluralité des réservoirs de stockage (1) afin de déterminer quel milieu liquide est le plus fréquemment utilisé.

13. Système de distribution de parfum (100) selon la revendication 1, comprenant en outre :
une ou plusieurs pompes (21) disposées entre la pluralité de réservoirs de stockage (1) et l'un ou plusieurs dispositifs de distribution (2), dans lequel l'une ou plusieurs pompes (21) sont adaptées pour pomper le milieu liquide à partir des réservoirs de stockage (1) vers l'un ou plusieurs dispositifs de distribution (2).

14. Système de distribution de parfum (100) selon la revendication 1, dans lequel chaque réservoir de la pluralité de réservoirs de stockage (1) est relié à l'un des dispositifs respectifs parmi un ou plusieurs dispositifs de distribution (2) par l'intermédiaire d'un tube (110, 130).
